# EUROPEAN PATENT APPLICATION

(11) **EP 1 767 657 A1**
(43) Date of publication of application: **28.03.2007**
(21) Application number: 06000085.8
(22) Date of filing: 03.01.2006
(51) Int. Cl.: C12Q 1/70

(54) **Detecting foot-and-mouth disease virus**

(30) Priority: 21.09.2005 US 233245
(71) Applicant: Fair Isaac Corporation, San Diego CA 92130 (US)
(72) Inventor: Engelhard, Eric K., Davis, California 95616 (US)
(74) Representative: Grund, Martin

(57) **Abstract**

This document relates to methods and materials involved in determining whether or not an animal contains a foot-and-mouth disease virus. For example, nucleic acid primer pairs, combinations of nucleic acid primer pairs, nucleic acid arrays (e.g., diagnostic cards) containing nucleic acid primer pairs or combinations of nucleic acid primer pairs, methods for making such nucleic acid arrays, and methods for diagnosing animals infected with a foot-and-mouth disease virus are provided.

SEQUENCE LISTING

<110> Fair Isaac Corporation

<120> Detecting Foot-and-Mouth Disease Virus
<130> 116-30

<150> US 11/233,245
<151> 21 September 2005

<160> 227

<170> FastSEQ for Windows Version 4.0

<210> 1
<211> 27
<212> DNA
<213> Artificial Sequence

<220>
<223> fragment present in foot and mouth virus

<400> 1
cctactactt ctctgatttg gaaattg                                          27

<210> 2
<211> 25
<212> DNA
<213> Artificial Sequence

<220>
<223> fragment present in foot and mouth virus

<400> 2
tgtttgaacc actcacagtg tactt                                            25

<210> 3
<211> 25
<212> DNA
<213> Artificial Sequence

<220>
<223> fragment present in foot and mouth virus

<400> 3
aacaagtaca ctgtgagtgg ttcaa                                            25

<210> 4
<211> 26
<212> DNA
<213> Artificial Sequence

<220>
<223> fragment present in foot and mouth virus

<400> 4
aatgatcttc tgtttgtgtc tgtctt                                           26

<210> 5
<211> 25
<212> DNA
<213> Artificial Sequence

<220>
<223> fragment present in foot and mouth virus

<400> 5
caatgatctt ctgtttgtgt ctgtc                                          25

<210> 6
<211> 26
<212> DNA
<213> Artificial Sequence

<220>
<223> fragment present in foot and mouth virus

<400> 6
actatttctc tgacttggaa gttgtg                                         26

<210> 7
<211> 25
<212> DNA
<213> Artificial Sequence

<220>
<223> fragment present in foot and mouth virus

<400> 7
gtagttgaag gatgaaggga gttgt                                          25

<210> 8
<211> 25
<212> DNA
<213> Artificial Sequence

<220>
<223> fragment present in foot and mouth virus

<400> 8
tcagccacct actatttctc tgact                                          25

<210> 9
<211> 25
<212> DNA
<213> Artificial Sequence

<220>
<223> fragment present in foot and mouth virus

<400> 9
ttcagccacc tactatttct ctgac                                          25

<210> 10
<211> 25
<212> DNA
<213> Artificial Sequence

<220>
<223> fragment present in foot and mouth virus

<400> 10
ccacctacta tttctctgac ttgga                                          25

<210> 11
<211> 24
<212> DNA
<213> Artificial Sequence

<220>
<223> fragment present in foot and mouth virus

<400> 11
tagttgaagg atgaagggag ttgt                                           24

<210> 12
<211> 24
<212> DNA
<213> Artificial Sequence

<220>
<223> fragment present in foot and mouth virus

<400> 12
gtcaccacca ctgttgagaa ctac                                           24

<210> 13
<211> 27
<212> DNA
<213> Artificial Sequence

<220>
<223> fragment present in foot and mouth virus

<400> 13
caccacaatc tctaggtcag agaagta                                        27

<210> 14
<211> 27
<212> DNA
<213> Artificial Sequence

<220>
<223> fragment present in foot and mouth virus

<400> 14
tctctaggtc agagaagtag tacgtgg                                        27

<210> 15
<211> 26
<212> DNA
<213> Artificial Sequence

<220>
<223> fragment present in foot and mouth virus

<400> 15
ttctaggtca gagaagtagt acgtgg                                          26

<210> 16
<211> 25
<212> DNA
<213> Artificial Sequence

<220>
<223> fragment present in foot and mouth virus

<400> 16
gttgagaact acggaggaga aactc                                           25

<210> 17
<211> 25
<212> DNA
<213> Artificial Sequence

<220>
<223> fragment present in foot and mouth virus

<400> 17
caacctccag gtctgagaag tagta                                           25

<210> 18
<211> 25
<212> DNA
<213> Artificial Sequence

<220>
<223> fragment present in foot and mouth virus

<400> 18
aactacggag gagaaactca gacag                                           25

<210> 19
<211> 25
<212> DNA
<213> Artificial Sequence

<220>
<223> fragment present in foot and mouth virus

<400> 19
acctccaggt ctgagaagta gtacg                                           25

<210> 20
<211> 25
<212> DNA
<213> Artificial Sequence

<220>
<223> fragment present in foot and mouth virus

<400> 20
agaactacgg aggagaaact cagac                                           25

<210> 21

<211> 25
<212> DNA
<213> Artificial Sequence

<220>
<223> fragment present in foot and mouth virus

<400> 21
agttgagaac tacggaggag agact                                          25

<210> 22
<211> 24
<212> DNA
<213> Artificial Sequence

<220>
<223> fragment present in foot and mouth virus

<400> 22
aacctccagg tccgagaagt agta                                           24

<210> 23
<211> 25
<212> DNA
<213> Artificial Sequence

<220>
<223> fragment present in foot and mouth virus

<400> 23
cagttgagaa ctacggagga gagac                                          25

<210> 24
<211> 24
<212> DNA
<213> Artificial Sequence

<220>
<223> fragment present in foot and mouth virus

<400> 24
gtacactggc actacgacct acac                                           24

<210> 25
<211> 25
<212> DNA
<213> Artificial Sequence

<220>
<223> fragment present in foot and mouth virus

<400> 25
agaatcggcc taggacaata gagtt                                          25

<210> 26
<211> 24
<212> DNA
<213> Artificial Sequence

<220>
<223> fragment present in foot and mouth virus

<400> 26
agtttctgca cttgacaaca caac                                              24

<210> 27
<211> 26
<212> DNA
<213> Artificial Sequence

<220>
<223> fragment present in foot and mouth virus

<400> 27
aactcagtga ttgtttctgc tttaac                                            26

<210> 28
<211> 25
<212> DNA
<213> Artificial Sequence

<220>
<223> fragment present in foot and mouth virus

<400> 28
cagccacgta ctacttctct gatct                                             25

<210> 29
<211> 27
<212> DNA
<213> Artificial Sequence

<220>
<223> fragment present in foot and mouth virus

<400> 29
tagtgtaggt tgttgtacca gtgtacg                                           27

<210> 30
<211> 25
<212> DNA
<213> Artificial Sequence

<220>
<223> fragment present in foot and mouth virus

<400> 30
gtacacaagg acagtattgt gggag                                             25

<210> 31
<211> 26
<212> DNA
<213> Artificial Sequence

<220>
<223> fragment present in foot and mouth virus

<400> 31

actggtagtg taggttgttg taccag                                          26

<210> 32
<211> 26
<212> DNA
<213> Artificial Sequence

<220>
<223> fragment present in foot and mouth virus

<400> 32
ctggtagtgt aggttgttgt accagt                                          26

<210> 33
<211> 25
<212> DNA
<213> Artificial Sequence

<220>
<223> fragment present in foot and mouth virus

<400> 33
cacctactac ttctctgacc tggag                                           25

<210> 34
<211> 26
<212> DNA
<213> Artificial Sequence

<220>
<223> fragment present in foot and mouth virus

<400> 34
cttctgagct aacacttgaa ggtcac                                          26

<210> 35
<211> 25
<212> DNA
<213> Artificial Sequence

<220>
<223> fragment present in foot and mouth virus

<400> 35
agagttcttt ctgccttctg agcta                                           25

<210> 36
<211> 25
<212> DNA
<213> Artificial Sequence

<220>
<223> fragment present in foot and mouth virus

<400> 36
agttctttct gccttctgag ctaac                                           25

<210> 37
<211> 23

<212> DNA
<213> Artificial Sequence

<220>
<223> fragment present in foot and mouth virus

<400> 37
ctgtgaccaa tgtgagaggt gac                                              23

<210> 38
<211> 26
<212> DNA
<213> Artificial Sequence

<220>
<223> fragment present in foot and mouth virus

<400> 38
acaatctttt gtttgtgtct agcttc                                           26

<210> 39
<211> 24
<212> DNA
<213> Artificial Sequence

<220>
<223> fragment present in foot and mouth virus

<400> 39
tgtgagaggt gacctacaag tgtt                                             24

<210> 40
<211> 23
<212> DNA
<213> Artificial Sequence

<220>
<223> fragment present in foot and mouth virus

<400> 40
gtcttctgtt tgtttctggc ttc                                              23

<210> 41
<211> 25
<212> DNA
<213> Artificial Sequence

<220>
<223> fragment present in foot and mouth virus

<400> 41
tcatcatgga cagatttgtg aaagt                                            25

<210> 42
<211> 25
<212> DNA
<213> Artificial Sequence

<220>

<223> fragment present in foot and mouth virus

<400> 42
gtctccctcg tgttttactg ctatc                                          25

<210> 43
<211> 25
<212> DNA
<213> Artificial Sequence

<220>
<223> fragment present in foot and mouth virus

<400> 43
atcatggaca gatttgtgaa agtga                                          25

<210> 44
<211> 25
<212> DNA
<213> Artificial Sequence

<220>
<223> fragment present in foot and mouth virus

<400> 44
ctccctcgtg ttttactgct atctc                                          25

<210> 45
<211> 25
<212> DNA
<213> Artificial Sequence

<220>
<223> fragment present in foot and mouth virus

<400> 45
agatttgtga agattggaac cacta                                          25

<210> 46
<211> 23
<212> DNA
<213> Artificial Sequence

<220>
<223> fragment present in foot and mouth virus

<400> 46
gagtacttgt tcgtcccgtt gta                                            23

<210> 47
<211> 23
<212> DNA
<213> Artificial Sequence

<220>
<223> fragment present in foot and mouth virus

<400> 47
caacttcctg cctctttcaa ttt                                            23

<210> 48
<211> 28
<212> DNA
<213> Artificial Sequence

<220>
<223> fragment present in foot and mouth virus

<400> 48
acttactact tcgctgattt agaagtgg                                          28

<210> 49
<211> 23
<212> DNA
<213> Artificial Sequence

<220>
<223> fragment present in foot and mouth virus

<400> 49
ctagcacctg gagatcacct ctc                                               23

<210> 50
<211> 27
<212> DNA
<213> Artificial Sequence

<220>
<223> fragment present in foot and mouth virus

<400> 50
cttactactt cgctgattta gaagtgg                                           27

<210> 51
<211> 23
<212> DNA
<213> Artificial Sequence

<220>
<223> fragment present in foot and mouth virus

<400> 51
accgtagtta aaggaggtag gca                                               23

<210> 52
<211> 25
<212> DNA
<213> Artificial Sequence

<220>
<223> fragment present in foot and mouth virus

<400> 52
gagttgcaag tacagcagag ttgag                                             25

<210> 53
<211> 23
<212> DNA

<213> Artificial Sequence

<220>
<223> fragment present in foot and mouth virus

<400> 53
caagagttgt ttcataggtg cca                                              23

<210> 54
<211> 24
<212> DNA
<213> Artificial Sequence

<220>
<223> fragment present in foot and mouth virus

<400> 54
agttgcaagt acagcagagt tgag                                             24

<210> 55
<211> 26
<212> DNA
<213> Artificial Sequence

<220>
<223> fragment present in foot and mouth virus

<400> 55
ctttgatagc agtaaaagga gacgtt                                           26

<210> 56
<211> 24
<212> DNA
<213> Artificial Sequence

<220>
<223> fragment present in foot and mouth virus

<400> 56
aagtctcaag ttgggagcat ttct                                             24

<210> 57
<211> 25
<212> DNA
<213> Artificial Sequence

<220>
<223> fragment present in foot and mouth virus

<400> 57
tctttgatag cagtaaaagg agacg                                            25

<210> 58
<211> 23
<212> DNA
<213> Artificial Sequence

<220>
<223> fragment present in foot and mouth virus

<400> 58
agtctcaagt tgggagcatt tct                                              23

<210> 59
<211> 23
<212> DNA
<213> Artificial Sequence

<220>
<223> fragment present in foot and mouth virus

<400> 59
aacacacgga cgtctcattc ata                                              23

<210> 60
<211> 28
<212> DNA
<213> Artificial Sequence

<220>
<223> fragment present in foot and mouth virus

<400> 60
acttctaagt cagcgaaata gtaagtgg                                         28

<210> 61
<211> 28
<212> DNA
<213> Artificial Sequence

<220>
<223> fragment present in foot and mouth virus

<400> 61
acttactatt tcgctgactt agaagtgg                                         28

<210> 62
<211> 23
<212> DNA
<213> Artificial Sequence

<220>
<223> fragment present in foot and mouth virus

<400> 62
gtacttgcag ttcccgttgt aaa                                              23

<210> 63
<211> 27
<212> DNA
<213> Artificial Sequence

<220>
<223> fragment present in foot and mouth virus

<400> 63
tactgctact tactacttcg cagacct                                          27

<210> 64
<211> 23
<212> DNA
<213> Artificial Sequence

<220>
<223> fragment present in foot and mouth virus

<400> 64
ctaacacttg caggtcacct ctc                                               23

<210> 65
<211> 27
<212> DNA
<213> Artificial Sequence

<220>
<223> fragment present in foot and mouth virus

<400> 65
actgctactt actacttcgc agaccta                                           27

<210> 66
<211> 23
<212> DNA
<213> Artificial Sequence

<220>
<223> fragment present in foot and mouth virus

<400> 66
ccgttgtaaa cagtagccaa cac                                               23

<210> 67
<211> 27
<212> DNA
<213> Artificial Sequence

<220>
<223> fragment present in foot and mouth virus

<400> 67
agatttgtga aagtaacacc aaaagac                                           27

<210> 68
<211> 24
<212> DNA
<213> Artificial Sequence

<220>
<223> fragment present in foot and mouth virus

<400> 68
cgttgtaaac agtagccaag acac                                              24

<210> 69
<211> 27
<212> DNA
<213> Artificial Sequence

<220>
<223> fragment present in foot and mouth virus

<400> 69
ttagacagat ttgtgaaagt aacacca                                          27

<210> 70
<211> 24
<212> DNA
<213> Artificial Sequence

<220>
<223> fragment present in foot and mouth virus

<400> 70
gttgtaaaca gtagccaaga cacg                                             24

<210> 71
<211> 24
<212> DNA
<213> Artificial Sequence

<220>
<223> fragment present in foot and mouth virus

<400> 71
gtttacaacg ggaactgcaa gtat                                             24

<210> 72
<211> 25
<212> DNA
<213> Artificial Sequence

<220>
<223> fragment present in foot and mouth virus

<400> 72
cgtcagaaac ctcttaaagt gaaag                                            25

<210> 73
<211> 23
<212> DNA
<213> Artificial Sequence

<220>
<223> fragment present in foot and mouth virus

<400> 73
ctcagtgacg atcaagttct ttg                                              23

<210> 74
<211> 23
<212> DNA
<213> Artificial Sequence

<220>
<223> fragment present in foot and mouth virus

<400> 74
atctcaattc cttcccaaaa gtc                                              23

<210> 75
<211> 25
<212> DNA
<213> Artificial Sequence

<220>
<223> fragment present in foot and mouth virus

<400> 75
tgatgtttgc tttctcaatg tactc                                            25

<210> 76
<211> 25
<212> DNA
<213> Artificial Sequence

<220>
<223> fragment present in foot and mouth virus

<400> 76
aaagtgacac caaaagacca aatta                                            25

<210> 77
<211> 24
<212> DNA
<213> Artificial Sequence

<220>
<223> fragment present in foot and mouth virus

<400> 77
cgttgtagac agtagccaaa acac                                             24

<210> 78
<211> 23
<212> DNA
<213> Artificial Sequence

<220>
<223> fragment present in foot and mouth virus

<400> 78
accgtgtctt ggctactgtt tac                                              23

<210> 79
<211> 24
<212> DNA
<213> Artificial Sequence

<220>
<223> fragment present in foot and mouth virus

<400> 79
cttttgtttg tgtctagctt cgct                                             24

<210> 80

<211> 23
<212> DNA
<213> Artificial Sequence

<220>
<223> fragment present in foot and mouth virus

<400> 80
ttactcgact tgccttgcct tac                                              23

<210> 81
<211> 23
<212> DNA
<213> Artificial Sequence

<220>
<223> fragment present in foot and mouth virus

<400> 81
ctatcttctg tttgtgcctg gct                                              23

<210> 82
<211> 25
<212> DNA
<213> Artificial Sequence

<220>
<223> fragment present in foot and mouth virus

<400> 82
ctcagtgact atcaagttct ttgct                                            25

<210> 83
<211> 25
<212> DNA
<213> Artificial Sequence

<220>
<223> fragment present in foot and mouth virus

<400> 83
agaaacctct taaagtgaaa gccag                                            25

<210> 84
<211> 26
<212> DNA
<213> Artificial Sequence

<220>
<223> fragment present in foot and mouth virus

<400> 84
ctcagtgact atcaagttct ttgctt                                           26

<210> 85
<211> 26
<212> DNA
<213> Artificial Sequence

<220>
<223> fragment present in foot and mouth virus

<400> 85
ttagacttgc tcaagacaaa agagaa                                            26

<210> 86
<211> 24
<212> DNA
<213> Artificial Sequence

<220>
<223> fragment present in foot and mouth virus

<400> 86
ttgtacttgc aatcaccgtt gtag                                              24

<210> 87
<211> 26
<212> DNA
<213> Artificial Sequence

<220>
<223> fragment present in foot and mouth virus

<400> 87
gttagacttg ctcaagacaa aagaga                                            26

<210> 88
<211> 24
<212> DNA
<213> Artificial Sequence

<220>
<223> fragment present in foot and mouth virus

<400> 88
cttgtacttg caatcaccgt tgta                                              24

<210> 89
<211> 25
<212> DNA
<213> Artificial Sequence

<220>
<223> fragment present in foot and mouth virus

<400> 89
acaacaagat ggtgttagac ttgct                                             25

<210> 90
<211> 24
<212> DNA
<213> Artificial Sequence

<220>
<223> fragment present in foot and mouth virus

<400> 90

gtacttgcaa tcaccgttgt aggt                                          24

<210> 91
<211> 25
<212> DNA
<213> Artificial Sequence

<220>
<223> fragment present in foot and mouth virus

<400> 91
caggtgtctt gcaacaactt acaat                                         25

<210> 92
<211> 25
<212> DNA
<213> Artificial Sequence

<220>
<223> fragment present in foot and mouth virus

<400> 92
aggttttgtt atcgctgtct tgtag                                         25

<210> 93
<211> 25
<212> DNA
<213> Artificial Sequence

<220>
<223> fragment present in foot and mouth virus

<400> 93
aggtgtcttg caacaactta caatg                                         25

<210> 94
<211> 25
<212> DNA
<213> Artificial Sequence

<220>
<223> fragment present in foot and mouth virus

<400> 94
gtaaatcctg ccgtagttaa aagtg                                         25

<210> 95
<211> 25
<212> DNA
<213> Artificial Sequence

<220>
<223> fragment present in foot and mouth virus

<400> 95
acacaggtgt cttgcaacaa cttac                                         25

<210> 96
<211> 25

<212> DNA
<213> Artificial Sequence

<220>
<223> fragment present in foot and mouth virus

<400> 96
tggtgactgt aagtacaaac ccact                                          25

<210> 97
<211> 24
<212> DNA
<213> Artificial Sequence

<220>
<223> fragment present in foot and mouth virus

<400> 97
cagctttaac aggtcgaaat taca                                           24

<210> 98
<211> 25
<212> DNA
<213> Artificial Sequence

<220>
<223> fragment present in foot and mouth virus

<400> 98
caacatccta caatggtgac tgtaa                                          25

<210> 99
<211> 26
<212> DNA
<213> Artificial Sequence

<220>
<223> fragment present in foot and mouth virus

<400> 99
tacacaactg tttgacaggc ttagtt                                         26

<210> 100
<211> 23
<212> DNA
<213> Artificial Sequence

<220>
<223> fragment present in foot and mouth virus

<400> 100
gtcttctcca aaaacaacac cac                                            23

<210> 101
<211> 26
<212> DNA
<213> Artificial Sequence

<220>

<223> fragment present in foot and mouth virus

<400> 101
gtgtgtaaag cctgccatag ttaaag                                          26

<210> 102
<211> 23
<212> DNA
<213> Artificial Sequence

<220>
<223> fragment present in foot and mouth virus

<400> 102
agtcgtcttc tccaaaaaca aca                                             23

<210> 103
<211> 27
<212> DNA
<213> Artificial Sequence

<220>
<223> fragment present in foot and mouth virus

<400> 103
actttcaact acggtaggat ctacaca                                         27

<210> 104
<211> 24
<212> DNA
<213> Artificial Sequence

<220>
<223> fragment present in foot and mouth virus

<400> 104
aactttaaca ggtcgaagtt gcac                                            24

<210> 105
<211> 27
<212> DNA
<213> Artificial Sequence

<220>
<223> fragment present in foot and mouth virus

<400> 105
ctttcaacta cggtaggatc tacacag                                         27

<210> 106
<211> 23
<212> DNA
<213> Artificial Sequence

<220>
<223> fragment present in foot and mouth virus

<400> 106
tcctacaacg gtgactgcaa gta                                             23

<210> 107
<211> 25
<212> DNA
<213> Artificial Sequence

<220>
<223> fragment present in foot and mouth virus

<400> 107
gtgatcgtag tgtgtgagaa gaggt                                          25

<210> 108
<211> 26
<212> DNA
<213> Artificial Sequence

<220>
<223> fragment present in foot and mouth virus

<400> 108
aactgtctac aacggtgagt gtaaat                                         26

<210> 109
<211> 25
<212> DNA
<213> Artificial Sequence

<220>
<223> fragment present in foot and mouth virus

<400> 109
tcatcctgta gtacacgtca acact                                          25

<210> 110
<211> 25
<212> DNA
<213> Artificial Sequence

<220>
<223> fragment present in foot and mouth virus

<400> 110
catcctgtag tacacgtcaa cactt                                          25

<210> 111
<211> 25
<212> DNA
<213> Artificial Sequence

<220>
<223> fragment present in foot and mouth virus

<400> 111
tgagagcttc cacctactac ttctg                                          25

<210> 112
<211> 26
<212> DNA

<213> Artificial Sequence

<220>
<223> fragment present in foot and mouth virus

<400> 112
tgtgtattta cactcaccgt tgtaga                                          26

<210> 113
<211> 27
<212> DNA
<213> Artificial Sequence

<220>
<223> fragment present in foot and mouth virus

<400> 113
cactgtttac aacggtgagt gtaagta                                         27

<210> 114
<211> 25
<212> DNA
<213> Artificial Sequence

<220>
<223> fragment present in foot and mouth virus

<400> 114
gcatagttgt ttctctaccc caata                                           25

<210> 115
<211> 25
<212> DNA
<213> Artificial Sequence

<220>
<223> fragment present in foot and mouth virus

<400> 115
cactgtttac aacggtgagt gtaag                                           25

<210> 116
<211> 26
<212> DNA
<213> Artificial Sequence

<220>
<223> fragment present in foot and mouth virus

<400> 116
gcatagttgt ttctctaccc caatag                                          26

<210> 117
<211> 26
<212> DNA
<213> Artificial Sequence

<220>
<223> fragment present in foot and mouth virus

<400> 117
taacacaact gtacaaccaa tacgtg                                          26

<210> 118
<211> 25
<212> DNA
<213> Artificial Sequence

<220>
<223> fragment present in foot and mouth virus

<400> 118
ccacctacta tttttgtgac ttgga                                           25

<210> 119
<211> 25
<212> DNA
<213> Artificial Sequence

<220>
<223> fragment present in foot and mouth virus

<400> 119
tggagtagtt acactcaccg ttgta                                           25

<210> 120
<211> 25
<212> DNA
<213> Artificial Sequence

<220>
<223> fragment present in foot and mouth virus

<400> 120
tatttttgtg acttggaaat tgcat                                           25

<210> 121
<211> 24
<212> DNA
<213> Artificial Sequence

<220>
<223> fragment present in foot and mouth virus

<400> 121
agcactttca actacggtta cgtg                                            24

<210> 122
<211> 24
<212> DNA
<213> Artificial Sequence

<220>
<223> fragment present in foot and mouth virus

<400> 122
acacagttgt tttctacgc caat                                             24

<210> 123
<211> 26
<212> DNA
<213> Artificial Sequence

<220>
<223> fragment present in foot and mouth virus

<400> 123
gtactacttt gctgaccttg aaatcg                                          26

<210> 124
<211> 24
<212> DNA
<213> Artificial Sequence

<220>
<223> fragment present in foot and mouth virus

<400> 124
acgtaaccgt agttgaaagt gctg                                            24

<210> 125
<211> 24
<212> DNA
<213> Artificial Sequence

<220>
<223> fragment present in foot and mouth virus

<400> 125
gtttacaacg gtgaatgcaa atac                                            24

<210> 126
<211> 26
<212> DNA
<213> Artificial Sequence

<220>
<223> fragment present in foot and mouth virus

<400> 126
atcctgtagt acacatcaac gctact                                          26

<210> 127
<211> 26
<212> DNA
<213> Artificial Sequence

<220>
<223> fragment present in foot and mouth virus

<400> 127
tgtagtacac atcaacgcta ctgtca                                          26

<210> 128
<211> 25
<212> DNA
<213> Artificial Sequence

<220>
<223> fragment present in foot and mouth virus

<400> 128
caaagcagtt gatgtgtact acagg                                          25

<210> 129
<211> 25
<212> DNA
<213> Artificial Sequence

<220>
<223> fragment present in foot and mouth virus

<400> 129
caactttaac aggtcgtagt tgcac                                          25

<210> 130
<211> 25
<212> DNA
<213> Artificial Sequence

<220>
<223> fragment present in foot and mouth virus

<400> 130
acattcaact ttggtcacgt tactg                                          25

<210> 131
<211> 23
<212> DNA
<213> Artificial Sequence

<220>
<223> fragment present in foot and mouth virus

<400> 131
ccagcacttt caactacggt tac                                            23

<210> 132
<211> 23
<212> DNA
<213> Artificial Sequence

<220>
<223> fragment present in foot and mouth virus

<400> 132
gcactttcaa ctacggttac gtg                                            23

<210> 133
<211> 26
<212> DNA
<213> Artificial Sequence

<220>
<223> fragment present in foot and mouth virus

<400> 133
caactttaac aggtcgaagt tacaca                                          26

<210> 134
<211> 25
<212> DNA
<213> Artificial Sequence

<220>
<223> fragment present in foot and mouth virus

<400> 134
ctatcaacca gatacaacgg tgagt                                           25

<210> 135
<211> 26
<212> DNA
<213> Artificial Sequence

<220>
<223> fragment present in foot and mouth virus

<400> 135
aggagaggtc ttggacagta gagttc                                          26

<210> 136
<211> 25
<212> DNA
<213> Artificial Sequence

<220>
<223> fragment present in foot and mouth virus

<400> 136
ctgtctactg tctacaatgg cgagt                                           25

<210> 137
<211> 25
<212> DNA
<213> Artificial Sequence

<220>
<223> fragment present in foot and mouth virus

<400> 137
cttcatccgg tagtaaacat cgact                                           25

<210> 138
<211> 25
<212> DNA
<213> Artificial Sequence

<220>
<223> fragment present in foot and mouth virus

<400> 138
aggctgctgt ctactgtcta caatg                                           25

<210> 139

<211> 24
<212> DNA
<213> Artificial Sequence

<220>
<223> fragment present in foot and mouth virus

<400> 139
acagtacagt tcagccctct tcat                                              24

<210> 140
<211> 25
<212> DNA
<213> Artificial Sequence

<220>
<223> fragment present in foot and mouth virus

<400> 140
cagtgttctt gcaacagtct acaat                                             25

<210> 141
<211> 28
<212> DNA
<213> Artificial Sequence

<220>
<223> fragment present in foot and mouth virus

<400> 141
aattacacag ttgtttatca ggtgctac                                          28

<210> 142
<211> 25
<212> DNA
<213> Artificial Sequence

<220>
<223> fragment present in foot and mouth virus

<400> 142
gtctacaatg gcaactgcaa atact                                             25

<210> 143
<211> 25
<212> DNA
<213> Artificial Sequence

<220>
<223> fragment present in foot and mouth virus

<400> 143
gttctcttca tccggtagta gacct                                             25

<210> 144
<211> 25
<212> DNA
<213> Artificial Sequence

<220>
<223> fragment present in foot and mouth virus

<400> 144
ttctcttcat ccggtagtag acctc                                          25

<210> 145
<211> 25
<212> DNA
<213> Artificial Sequence

<220>
<223> fragment present in foot and mouth virus

<400> 145
acttcaacag gtcgaaatta cacag                                          25

<210> 146
<211> 23
<212> DNA
<213> Artificial Sequence

<220>
<223> fragment present in foot and mouth virus

<400> 146
ctgtaaaggc tgacaccatc act                                            23

<210> 147
<211> 25
<212> DNA
<213> Artificial Sequence

<220>
<223> fragment present in foot and mouth virus

<400> 147
tcaggtgcaa tgatcttctg tttac                                          25

<210> 148
<211> 24
<212> DNA
<213> Artificial Sequence

<220>
<223> fragment present in foot and mouth virus

<400> 148
caggtgcaat gatcttctgt ttac                                           24

<210> 149
<211> 25
<212> DNA
<213> Artificial Sequence

<220>
<223> fragment present in foot and mouth virus

<400> 149

gttcttgaca ggtttgtgaa actca 25

<210> 150
<211> 23
<212> DNA
<213> Artificial Sequence

<220>
<223> fragment present in foot and mouth virus

<400> 150
gatcaaaagc tcagtgatgg tgt 23

<210> 151
<211> 23
<212> DNA
<213> Artificial Sequence

<220>
<223> fragment present in foot and mouth virus

<400> 151
atcaaaagct cagtgatggt gtc 23

<210> 152
<211> 23
<212> DNA
<213> Artificial Sequence

<220>
<223> fragment present in foot and mouth virus

<400> 152
acctctttca actacggtgc tgt 23

<210> 153
<211> 23
<212> DNA
<213> Artificial Sequence

<220>
<223> fragment present in foot and mouth virus

<400> 153
cataatctgc ttctcaggtg caa 23

<210> 154
<211> 25
<212> DNA
<213> Artificial Sequence

<220>
<223> fragment present in foot and mouth virus

<400> 154
tcttgacagg tttgtgaaac tcact 25

<210> 155
<211> 23

<212> DNA
<213> Artificial Sequence

<220>
<223> fragment present in foot and mouth virus

<400> 155
tttcaactac ggtgctgtaa agg                                              23

<210> 156
<211> 24
<212> DNA
<213> Artificial Sequence

<220>
<223> fragment present in foot and mouth virus

<400> 156
gttcttgaca ggtttgtgaa actc                                             24

<210> 157
<211> 24
<212> DNA
<213> Artificial Sequence

<220>
<223> fragment present in foot and mouth virus

<400> 157
cttgacaggt ttgtgaaact cact                                             24

<210> 158
<211> 24
<212> DNA
<213> Artificial Sequence

<220>
<223> fragment present in foot and mouth virus

<400> 158
ctagacaacc agaccaatcc aact                                             24

<210> 159
<211> 26
<212> DNA
<213> Artificial Sequence

<220>
<223> fragment present in foot and mouth virus

<400> 159
ctgagtagtg tcaagagcta gcaaag                                           26

<210> 160
<211> 23
<212> DNA
<213> Artificial Sequence

<220>

<223> fragment present in foot and mouth virus

<400> 160
tagacaacca gaccaatcca act                                              23

<210> 161
<211> 23
<212> DNA
<213> Artificial Sequence

<220>
<223> fragment present in foot and mouth virus

<400> 161
ctagacaacc agaccaatcc aac                                              23

<210> 162
<211> 23
<212> DNA
<213> Artificial Sequence

<220>
<223> fragment present in foot and mouth virus

<400> 162
acagtgtaca atgggaagac gac                                              23

<210> 163
<211> 23
<212> DNA
<213> Artificial Sequence

<220>
<223> fragment present in foot and mouth virus

<400> 163
ctctagacaa ccagaccaat cca                                              23

<210> 164
<211> 24
<212> DNA
<213> Artificial Sequence

<220>
<223> fragment present in foot and mouth virus

<400> 164
tctagacaac cagaccaatc caac                                             24

<210> 165
<211> 24
<212> DNA
<213> Artificial Sequence

<220>
<223> fragment present in foot and mouth virus

<400> 165
ctctagacaa ccagaccaat ccaa                                             24

<210> 166
<211> 23
<212> DNA
<213> Artificial Sequence

<220>
<223> fragment present in foot and mouth virus

<400> 166
tctagacaac cagaccaatc caa                                              23

<210> 167
<211> 23
<212> DNA
<213> Artificial Sequence

<220>
<223> fragment present in foot and mouth virus

<400> 167
gctctagaca accagaccaa tcc                                              23

<210> 168
<211> 25
<212> DNA
<213> Artificial Sequence

<220>
<223> fragment present in foot and mouth virus

<400> 168
agtagtgtca agagctagca aaggc                                            25

<210> 169
<211> 24
<212> DNA
<213> Artificial Sequence

<220>
<223> fragment present in foot and mouth virus

<400> 169
atgctctaga caaccagacc aatc                                             24

<210> 170
<211> 24
<212> DNA
<213> Artificial Sequence

<220>
<223> fragment present in foot and mouth virus

<400> 170
gatgctctag acaaccagac caat                                             24

<210> 171
<211> 23
<212> DNA

<213> Artificial Sequence

<220>
<223> fragment present in foot and mouth virus

<400> 171
tgctctagac aaccagacca atc                                              23

<210> 172
<211> 23
<212> DNA
<213> Artificial Sequence

<220>
<223> fragment present in foot and mouth virus

<400> 172
gatgctctag acaaccagac caa                                              23

<210> 173
<211> 23
<212> DNA
<213> Artificial Sequence

<220>
<223> fragment present in foot and mouth virus

<400> 173
atgctctaga caaccagacc aat                                              23

<210> 174
<211> 24
<212> DNA
<213> Artificial Sequence

<220>
<223> fragment present in foot and mouth virus

<400> 174
aaagatgctc tagacaacca gacc                                             24

<210> 175
<211> 25
<212> DNA
<213> Artificial Sequence

<220>
<223> fragment present in foot and mouth virus

<400> 175
gatcaaaagc tcagtaatgg tgtca                                            25

<210> 176
<211> 25
<212> DNA
<213> Artificial Sequence

<220>
<223> fragment present in foot and mouth virus

<400> 176
ctagacaacc aaactaaccc aactg                                          25

<210> 177
<211> 24
<212> DNA
<213> Artificial Sequence

<220>
<223> fragment present in foot and mouth virus

<400> 177
gagtggtgtc aagagctagc aaag                                           24

<210> 178
<211> 24
<212> DNA
<213> Artificial Sequence

<220>
<223> fragment present in foot and mouth virus

<400> 178
caaagatgct ctagacaacc agac                                           24

<210> 179
<211> 25
<212> DNA
<213> Artificial Sequence

<220>
<223> fragment present in foot and mouth virus

<400> 179
cctagacaac caaactaacc caact                                          25

<210> 180
<211> 25
<212> DNA
<213> Artificial Sequence

<220>
<223> fragment present in foot and mouth virus

<400> 180
atatggctgc ccttacacta aagac                                          25

<210> 181
<211> 23
<212> DNA
<213> Artificial Sequence

<220>
<223> fragment present in foot and mouth virus

<400> 181
gtgtactggc gacagtgtac aag                                            23

<210> 182
<211> 24
<212> DNA
<213> Artificial Sequence

<220>
<223> fragment present in foot and mouth virus

<400> 182
atatggctgc ccttacacta aaga                                              24

<210> 183
<211> 24
<212> DNA
<213> Artificial Sequence

<220>
<223> fragment present in foot and mouth virus

<400> 183
gatatggctg cccttacact aaag                                              24

<210> 184
<211> 23
<212> DNA
<213> Artificial Sequence

<220>
<223> fragment present in foot and mouth virus

<400> 184
gtgtactggc gacggtatac aac                                               23

<210> 185
<211> 25
<212> DNA
<213> Artificial Sequence

<220>
<223> fragment present in foot and mouth virus

<400> 185
tatggctgcc cttacactaa agact                                             25

<210> 186
<211> 24
<212> DNA
<213> Artificial Sequence

<220>
<223> fragment present in foot and mouth virus

<400> 186
aatcagacca atccaactgc ttac                                              24

<210> 187
<211> 24
<212> DNA
<213> Artificial Sequence

<220>
<223> fragment present in foot and mouth virus

<400> 187
gacagtgtac aacgggaaga ctac                                              24

<210> 188
<211> 24
<212> DNA
<213> Artificial Sequence

<220>
<223> fragment present in foot and mouth virus

<400> 188
tatggctgcc cttacactaa agac                                              24

<210> 189
<211> 25
<212> DNA
<213> Artificial Sequence

<220>
<223> fragment present in foot and mouth virus

<400> 189
gatatggctg cccttacact aaaga                                             25

<210> 190
<211> 23
<212> DNA
<213> Artificial Sequence

<220>
<223> fragment present in foot and mouth virus

<400> 190
aatcagacca atccaactgc tta                                               23

<210> 191
<211> 23
<212> DNA
<213> Artificial Sequence

<220>
<223> fragment present in foot and mouth virus

<400> 191
tatggctgcc cttacactaa aga                                               23

<210> 192
<211> 23
<212> DNA
<213> Artificial Sequence

<220>
<223> fragment present in foot and mouth virus

<400> 192
agactacgta cggggaaaca act                                                23

<210> 193
<211> 24
<212> DNA
<213> Artificial Sequence

<220>
<223> fragment present in foot and mouth virus

<400> 193
aagactacgt acggggaaac aact                                               24

<210> 194
<211> 23
<212> DNA
<213> Artificial Sequence

<220>
<223> fragment present in foot and mouth virus

<400> 194
atcagaccaa tccaactgct tac                                                23

<210> 195
<211> 24
<212> DNA
<213> Artificial Sequence

<220>
<223> fragment present in foot and mouth virus

<400> 195
agactacgta cggggaaaca actt                                               24

<210> 196
<211> 23
<212> DNA
<213> Artificial Sequence

<220>
<223> fragment present in foot and mouth virus

<400> 196
gatatggctg cccttacact aaa                                                23

<210> 197
<211> 23
<212> DNA
<213> Artificial Sequence

<220>
<223> fragment present in foot and mouth virus

<400> 197
acagtgtaca acggaaagac gac                                                23

<210> 198

<211> 23
<212> DNA
<213> Artificial Sequence

<220>
<223> fragment present in foot and mouth virus

<400> 198
actacgtacg gggaaacaac ttc                                              23

<210> 199
<211> 23
<212> DNA
<213> Artificial Sequence

<220>
<223> fragment present in foot and mouth virus

<400> 199
aagactacgt acggggaaac aac                                              23

<210> 200
<211> 24
<212> DNA
<213> Artificial Sequence

<220>
<223> fragment present in foot and mouth virus

<400> 200
aacagtgtac aacggaaaga cgac                                             24

<210> 201
<211> 23
<212> DNA
<213> Artificial Sequence

<220>
<223> fragment present in foot and mouth virus

<400> 201
gactacgtac ggggaaacaa ctt                                              23

<210> 202
<211> 23
<212> DNA
<213> Artificial Sequence

<220>
<223> fragment present in foot and mouth virus

<400> 202
aaagatgctc tggacaacca aac                                              23

<210> 203
<211> 24
<212> DNA
<213> Artificial Sequence

<220>
<223> fragment present in foot and mouth virus

<400> 203
atggctgccc ttacactaaa gact                                              24

<210> 204
<211> 24
<212> DNA
<213> Artificial Sequence

<220>
<223> fragment present in foot and mouth virus

<400> 204
acagtgtaca acgggaagac tacg                                              24

<210> 205
<211> 26
<212> DNA
<213> Artificial Sequence

<220>
<223> fragment present in foot and mouth virus

<400> 205
atatggctgc ccttacacta aagact                                            26

<210> 206
<211> 23
<212> DNA
<213> Artificial Sequence

<220>
<223> fragment present in foot and mouth virus

<400> 206
atggctgccc ttacactaaa gac                                               23

<210> 207
<211> 23
<212> DNA
<213> Artificial Sequence

<220>
<223> fragment present in foot and mouth virus

<400> 207
aacagtgtac aacggaaaga cga                                               23

<210> 208
<211> 24
<212> DNA
<213> Artificial Sequence

<220>
<223> fragment present in foot and mouth virus

<400> 208

tgatatggct gcccttacac taaa 24

<210> 209
<211> 23
<212> DNA
<213> Artificial Sequence

<220>
<223> fragment present in foot and mouth virus

<400> 209
gcaacagtgt acaacggaaa gac 23

<210> 210
<211> 23
<212> DNA
<213> Artificial Sequence

<220>
<223> fragment present in foot and mouth virus

<400> 210
cagtgtacaa cgggaagact acg 23

<210> 211
<211> 23
<212> DNA
<213> Artificial Sequence

<220>
<223> fragment present in foot and mouth virus

<400> 211
atatggctgc ccttacacta aag 23

<210> 212
<211> 23
<212> DNA
<213> Artificial Sequence

<220>
<223> fragment present in foot and mouth virus

<400> 212
gtgtacaacg gaaagacgac gta 23

<210> 213
<211> 23
<212> DNA
<213> Artificial Sequence

<220>
<223> fragment present in foot and mouth virus

<400> 213
gacagtgtac aacggaaaga cga 23

<210> 214
<211> 23

<212> DNA
<213> Artificial Sequence

<220>
<223> fragment present in foot and mouth virus

<400> 214
tggctgccct tacactaaag act                                              23

<210> 215
<211> 23
<212> DNA
<213> Artificial Sequence

<220>
<223> fragment present in foot and mouth virus

<400> 215
gaagactacg tacggggaaa caa                                              23

<210> 216
<211> 23
<212> DNA
<213> Artificial Sequence

<220>
<223> fragment present in foot and mouth virus

<400> 216
aagacgacgt acggaaaaca aac                                              23

<210> 217
<211> 23
<212> DNA
<213> Artificial Sequence

<220>
<223> fragment present in foot and mouth virus

<400> 217
gacagtgtac aacgggaaga cta                                              23

<210> 218
<211> 23
<212> DNA
<213> Artificial Sequence

<220>
<223> fragment present in foot and mouth virus

<400> 218
atccaactgc ctaccagaaa cag                                              23

<210> 219
<211> 23
<212> DNA
<213> Artificial Sequence

<220>

<223> fragment present in foot and mouth virus

<400> 219
acagtgtaca aggggaagac gac                                              23

<210> 220
<211> 23
<212> DNA
<213> Artificial Sequence

<220>
<223> fragment present in foot and mouth virus

<400> 220
caacagtgta caacggaaag acg                                              23

<210> 221
<211> 25
<212> DNA
<213> Artificial Sequence

<220>
<223> fragment present in foot and mouth virus

<400> 221
atcaaaagct cagtaatggt gtcag                                            25

<210> 222
<211> 23
<212> DNA
<213> Artificial Sequence

<220>
<223> fragment present in foot and mouth virus

<400> 222
atgctctaga caaccagacc aac                                              23

<210> 223
<211> 24
<212> DNA
<213> Artificial Sequence

<220>
<223> fragment present in foot and mouth virus

<400> 223
agatgctcta gacaaccaga ccaa                                             24

<210> 224
<211> 24
<212> DNA
<213> Artificial Sequence

<220>
<223> fragment present in foot and mouth virus

<400> 224
ccaaagatgc tctagacaac caga                                             24

<210> 225
<211> 23
<212> DNA
<213> Artificial Sequence

<220>
<223> fragment present in foot and mouth virus

<400> 225
actgactacc agaagcaacc cat                                              23

<210> 226
<211> 25
<212> DNA
<213> Artificial Sequence

<220>
<223> fragment present in foot and mouth virus

<400> 226
gagtagtgtc aagagctagc aaagg                                            25

<210> 227
<211> 25
<212> DNA
<213> Artificial Sequence

<220>
<223> fragment present in foot and mouth virus

<400> 227
tgagtagtgt caagagctag caaag                                            25

## Description

### BACKGROUND

### 1. Technical Field

This document relates to methods and materials involved in detecting foot-and-mouth disease virus in cloven-hooved animals (e.g., cattle).

### 2. Background Information

Cloven-hooved animals such as cattle can become infected with foot-and-mouth disease (FMD) viruses. In some cases, infected animals can become severely ill and even die because of an FMD virus infection. Typically, infected animals experience a lack of weight gain, reduced milk yield, and general un-thriftiness. Properly diagnosing infected animals can help to identify animals for treatment or vaccination and can help to control an outbreak of FMD within, for example, a herd, region, or zoo.

### SUMMARY

This document relates to methods and materials involved in detecting FMD viruses in cloven-hooved animals such as cattle, pigs, sheep, goats, buffalo, deer, and elephants. For example, this document provides nucleic acid primer pairs that can be used in an amplification reaction to detect the presence or absence of a FMD virus' nucleic acid within a sample obtained from the animal being tested. This document also provides combinations of nucleic acid primer pairs, nucleic acid arrays (e.g., diagnostic cards) containing nucleic acid primer pairs or combinations of nucleic acid primer pairs, methods for making such nucleic acid arrays, and methods for diagnosing animals infected with a FMD virus. Such methods and materials can allow, for example, cattle farmers to diagnose an animal as having an FMD virus infection. For example, the nucleic acid primer pairs provided herein can be used to diagnose a cow as having an FMD virus or as being free of FMD viruses. Once diagnosed as having an FMD virus infection, a veterinarian can identify proper treatments or procedures for the infected animal.

The description provided herein is based, in part, on the discovery of nucleic acid primer pairs having the ability to not only amplify particular nucleic acid sequences from FMD viruses, but also to not amplify nucleic acid sequences from non-FMD virus sources such as the host's genome. The description provided herein also is based, in part, on the discovery of sets of nucleic acid primer pairs that can be used simultaneously under the same amplification reaction conditions to amplify different target nucleic acids if present in the sample being tested. For example, a single diagnostic card having ten separate microfluidic chambers, each of which contains a different primer pair provided herein, can be used in a single amplification reaction to detect the presence or absence of up to ten different strains of FMD viruses. Having the ability to test for the presence or absence of multiple strains of FMD viruses using a single diagnostic card and a single amplification reaction can allow, for example, veterinarians to diagnose an animal's condition rapidly in a cost effective manner.

In general, one aspect of this document features a composition comprising, or consisting essentially of, a mixture, wherein the mixture comprises at least one (e.g., 1, 2, 3, 4, 5, 6, 7, 8, 9, 10, 20, 30, 40, 50, 60, 70, 80, 90, 100, 110, 120, 130, 140, 150, or more) primer pair selected from the group consisting of primer pair numbers 1-188 and 189 (e.g., 1, 2, 3, 4, 5, 6, 7, 8, 9, and 10), wherein the primer pair is capable of amplifying a sequence present in a foot-and-mouth disease virus. The mixture can be a solid. The mixture can be a liquid.

In another aspect, this document features an article of manufacture comprising, or consisting essentially of: (a) a substrate defining a microfluidic chamber and (b) a mixture comprising at least one (e.g., 1, 2, 3, 4, 5, 6, 7, 8, 9, 10, 20, 30, 40, 50, 60, 70, 80, 90, 100, 110, 120, 130, 140, 150, or more) primer pair selected from the group consisting of primer pair numbers 1-188 and 189 (e.g., 1, 2, 3, 4, 5, 6, 7, 8, 9, and 10); wherein the mixture is within the chamber; wherein the primer pair is capable of amplifying, within the chamber, a sequence present in a foot-and-mouth disease virus. The mixture can be a solid. The mixture can be a liquid.

In another aspect, this document features a diagnostic card for determining whether or not a cow contains a foot-and-mouth disease virus. The card comprises, or consists essentially of, a plurality of microfluidic chambers, wherein at least one of the microfluidic chambers comprises at least one (e.g., 1, 2, 3, 4, 5, 6, 7, 8, 9, 10, 20, 30, 40, 50, 60, 70, 80, 90, 100, 110, 120, 130, 140, 150, or more) primer pair selected from the group consisting of primer pair numbers 1-188 and 189 (e.g., 1, 2, 3, 4, 5, 6, 7, 8, 9, and 10), which are capable of amplifying, within the chamber, a sequence present in a foot-and-mouth disease virus.

In another aspect, this document features a method for determining whether or not a cloven-hooved animal contains a foot-and-mouth disease virus. The method comprises, or consists essentially of, performing an amplification reaction with at least one (e.g., 1, 2, 3, 4, 5, 6, 7, 8, 9, 10, 20, 30, 40, 50, 60, 70, 80, 90, 100, 110, 120, 130, 140, 150, or more) primer pair selected from the group consisting of primer pair numbers 1-188 and 189 (e.g., 1, 2, 3, 4, 5, 6, 7, 8, 9, and 10) to determine whether or not a sample from the animal contains nucleic acid capable of being amplified with the primer pair, wherein the presence of the nucleic acid indicates that the animal contains a foot-and-mouth disease virus. The animal can be a cow. The sample can be a blood sample.

In another aspect, this document features a method for making an article of manufacture for determining whether or not a cloven-hooved animal contains a foot-and-mouth disease virus. The method comprises, or consists essentially of, (a) providing a substrate defining a microfluidic chamber, and (b) placing a mixture into the chamber to form the article of manufacture, wherein the mixture comprises at least one (e.g., 1, 2, 3, 4, 5, 6, 7, 8, 9, 10, 20, 30, 40, 50, 60, 70, 80, 90, 100, 110, 120, 130, 140, 150, or more) primer pair selected from the group consisting of primer pair numbers 1-188 and 189 (e.g., 1, 2, 3, 4, 5, 6, 7, 8, 9, and 10); wherein the mixture is within the chamber; wherein the primer pair is capable of amplifying, within the chamber, a sequence present in a foot-and-mouth disease virus. The mixture can be a solid. The mixture can be a liquid. The animal can be a cow.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

Other features and advantages of the invention will be apparent from the following detailed description, and from the claims.

### DETAILED DESCRIPTION

This document relates to methods and materials involved in detecting FMD viruses in cloven-hooved animals such as cattle, pigs, sheep, goats, buffalo, deer, and elephants. For example, this document provides nucleic acid primer pairs that can be used in an amplification reaction to detect the presence or absence of an FMD virus' nucleic acid within a sample obtained from the animal being tested. This document also provides combinations of nucleic acid primer pairs, nucleic acid arrays (e.g., diagnostic cards) containing nucleic acid primer pairs or combinations of nucleic acid primer pairs, methods for making such nucleic acid arrays, and methods for diagnosing animals infected with an FMD virus.

Nucleic acid primer pairs provided herein are set forth in Table 1. Each primer pair can be used to amplify nucleic acid present in an FMD virus. For example, primer pair number 1 can be used to amplify nucleic acid present in an FMD virus, serotype A. Primer pair number 11 can be used to amplify nucleic acid present in an FMD virus, serotype Asia.

The nucleic acid primer pairs provided herein can be used separately or in combinations. Such combinations can contain 2, 3, 4, 5, 10, 15, 20, 25, 30, 35, 40, 45, 50, 55, 60, 70, 80, 90, 100, or more different nucleic acid primer pairs from Table 1. When making a combination, any two or more of the provided nucleic acid primer pairs can be arranged into any combination. For example, the first nucleic acid primer pair listed in Table 1 for each of the seven serotypes can be used to make a collection of seven different nucleic acid primer pairs. Other combinations include, without limitation, a combination of 89 different nucleic acid primer pairs containing the first 89 nucleic acid primer pairs listed in Table 1; a combination of 15 different nucleic acid primer pair containing the first 15 nucleic acid primer pairs listed in Table 1; and a combination of 100 different nucleic acid primer pairs containing the first 100 nucleic acid primer pairs listed in Table 1.

In some cases, the combination can contain nucleic acid primer pairs 1 through 89 listed in Table 1. Such a combination of nucleic acid primer pairs can be used to make a diagnostic card capable of diagnosing FMD virus infections found in cattle. Such diagnostic cards can be used to determine the presence or absence of an FMD virus within a sample. In some cases, such diagnostic cards can be used to identify a particular FMD virus' serotype and/or strain.

Each nucleic acid primer pair of a combination can be isolated from the other nucleic acid primer pairs of the combination. For example, each nucleic acid primer pair of a combination can be housed within a separate well of a plastic microtiter plate or a separate chamber of a microfluidic card. In some cases, each nucleic acid primer pair of a combination or a subset of nucleic acid primer pairs of a combination can be housed together. For example, five nucleic acid primer pairs of a combination of 50 nucleic acid primer pairs can be housed within a single well of a plastic microtiter plate with the remaining 45 nucleic acid primer pairs being housed within separate wells.

Any method can be used to make each nucleic acid primer of a nucleic acid primer pair. For example, chemical synthesis techniques such as those described elsewhere (Beaucage and Caruthers, Tetrahedron Lett., 22:1859-62 (1981)) can be used. In addition, nucleic acid primers can be ordered from commercial vendors such as MWG Biotech, Invitrogen, and Operon.

This description also provides arrays having at least one of the nucleic acid primer pairs provided herein. Such arrays can be any type of array including, without limitation, two-dimensional arrays, arrays in microtiter plates (e.g., plates with 48, 96, 384, or 1536 wells), arrays fabricated as an arrangement of microfluidic channels and chambers (e.g., a microfluidic card). In some cases, the array can be microfluidic cards with 8 loading ports each connected through microcapillaries to 48 reaction chambers. In some cases, an array provided herein can contain at least 10 different nucleic acid primer pairs set forth in Table 1 (e.g., at least 20, at least 30, at least 50, at least 100, or at least 200 different nucleic acid primer pairs set forth in Table 1).

In addition to containing any one or more of the nucleic acid primer pairs set forth in Table 1 in any combination, an array can contain nucleic acid primer pairs not listed in Table 1. For example, an array can contain a nucleic acid primer pair designed to amplify host nucleic acid (e.g., cattle genomic nucleic acid or mRNA). In some cases, at least 25% (e.g., at least 30%, at least 40%, at least 50%, at least 60%, at least 75%, at least 80%, at least 90%, at least 95%, or 100%) of the nucleic acid primer pairs of an array can be listed in Table 1.

The substrate of an array provided herein can be made of any suitable material (e.g., plastic, glass, silicone, or a metal). In addition, any method can be use to make an array. For example, spotted gelatine or photolithographic techniques can be used to make arrays. In some cases, an array provided herein can be made as follows. A 384-well master plate containing 125 µL of one or more primer pairs in dioinized water at a working concentration of 100 nmole/1 µL of each primer can be constructed. The master plate can be used as a template source, and 1 µL of each master plate well can be transferred to corresponding wells on a 384-well microfluidic card. Spotted reagents can be allowed to dry at room temperature before the final plastic laminate layer of the microfluidic card is attached.

As described herein, the nucleic acid primer pairs set forth in Table 1 can be used to determine whether or not a mammal (e.g., a cow) contains an FMD virus or a set of FMD viruses. For example, a sample can be obtained from a cow and used in an amplification reaction to determine whether or not an FMD virus' nucleic acid is present in the sample. The presence of an amplification product following an amplification reaction using an animal's blood sample and a nucleic acid primer pair designed for an FMD virus can indicate that that sample contains an FMD virus. In such a case, the animal can be diagnosed as being infected with an FMD virus. Any type of sample can be used including, without limitation, a biopsy (e.g., punch biopsy, aspiration biopsy, excision biopsy, needle biopsy, or shave biopsy), a tissue section, lymph fluid, blood, serum, saliva, anal swabs, and synovial fluid samples.

Some sample types can be pre-processed to enhance sample quality, such as the concentration of white blood cells through differential centrifugation. Samples can be processed to concentrate the nucleic acid and render it in a form to facilitate successful PCR reactions. This includes, but is not limited to, common methods to disrupt bilipid membranes, such as the use of detergents, digestion of protein complexes, such as the use of proteinase K, and reduction of polymerase inhibitors through the use of selective affinity columns. Commercial kits for purification of DNA, RNA, or total nucleic acid are readily available from, for example, Qiagen and Roche.

Any type of amplification reaction can be used in conjunction with the nucleic acid primer pairs set forth in Table 1 to detect an FMD virus. For example, common PCR reactions designed to amplify nucleic acid from DNA or RNA can be used. Detection of RNA viruses such as FMD viruses can be accomplished by synthesizing cDNA from RNA sequence templates. cDNA synthesis can be accomplished using standard methods using, for example, RNA-dependant DNA polymerases, such as reverse transcriptase. Such reactions can be primed with random oligonucleotide sequences, such as random hexamers and octamers, or by sequence specific oligonucleotide primers, including the same primers used for the PCR reaction. The cDNA synthesis can be performed in a separate reaction vessel from the subsequent PCR reaction (commonly referred to as two-step rtPCR) or in the same reaction vessel as the PCR reaction (commonly referred to as single-step rtPCR).

Purified DNA and cDNA samples can be pooled and added to a PCR master mix containing water, salt buffers, magnesium ions, nucleotide monomers (dATP, dCTP, dGTP and dTTP), native or engineered *Thermus aquaticus* DNA-dependant DNA polymerase, and an intercalating dye, such as Sybr Green or LC Green. The master mix and sample can then be added to a single loading port of a microfluidic card and dispersed to all the reaction wells using centrifugation. The cards can then be scored to isolate and seal each reaction chamber prior to thermocycling. The cards can be individually thermocycled using commodity block thermocyclers or many cards thermocycled simultaneously using air- or water-based thermocyclers such as the BioOven or the H2OBIT, respectively.

Positive PCR amplification reactions can be detected during thermocycling for quantitative or qualitative analysis (real time PCR) or after completion of thermocycling (qualitative end-point PCR). Signals can be detected through fluorescence-channel emission of substrate bound intercalating dyes using commodity real-time PCR capable PCR platforms or by end-point reads using microplate scanner platforms. Both types of platforms can be used for melting-point analysis for validation of positive signals.

**Table 1. Primer pairs that can be used to detect FMD viruses.**

| Primer Pair No. | FMDV Serotype | Forward Primer Sequence | SEQ ID NO: | Reverse Primer Sequence | SEQ ID NO: |
|---|---|---|---|---|---|
| 1 | A | CCTACTACTTCTCTGATTTGGAAATTG | 1 | TGTTTGAACCACTCACAGTGTACTT | 2 |
| 2 | A | AACAAGTACACTGTGAGTGGTTCAA | 3 | AATGATCTTCTGTTTGTGTCTGTCTT | 4 |
| 3 | A | AACAAGTACACTGTGAGTGGTTCAA | 3 | CAATGATCTTCTGTTTGTGTCTGTC | 5 |
| 4 | A | ACTATTTCTCTGACTTGGAAGTTGTG | 6 | GTAGTTGAAGGATGAAGGGAGTTGT | 7 |
| 5 | A | TCAGCCACCTACTATTTCTCTGACT | 8 | GTAGTTGAAGGATGAAGGGAGTTGT | 7 |
| 6 | A | TTCAGCCACCTACTATTTCTCTGAC | 9 | GTAGTTGAAGGATGAAGGGAGTTGT | 7 |
| 7 | A | CCACCTACTATTTCTCTGACTTGGA | 10 | TAGTTGAAGGATGAAGGGAGTTGT | 11 |
| 8 | A | GTCACCACCACTGTTGAGAACTAC | 12 | CACCACAATCTCTAGGTCAGAGAAGTA | 13 |
| 9 | A | GTCACCACCACTGTTGAGAACTAC | 12 | TCTCTAGGTCAGAGAAGTAGTACGTGG | 14 |
| 10 | A | GTCACCACCACTGTTGAGAACTAC | 12 | TTCTAGGTCAGAGAAGTAGTACGTGG | 15 |
| 11 | Asia | GTTGAGAACTACGGAGGAGAAACTC | 16 | CAACCTCCAGGTCTGAGAAGTAGTA | 17 |
| 12 | Asia | AACTACGGAGGAGAAACTCAGACAG | 18 | ACCTCCAGGTCTGAGAAGTAGTACG | 19 |
| 13 | Asia | AGAACTACGGAGGAGAAACTCAGAC | 20 | CAACCTCCAGGTCTGAGAAGTAGTA | 17 |
| 14 | Asia | AGTTGAGAACTACGGAGGAGAGACT | 21 | AACCTCCAGGTCCGAGAAGTAGTA | 22 |
| 15 | Asia | CAGTTGAGAACTACGGAGGAGAGAC | 23 | AACCTCCAGGTCCGAGAAGTAGTA | 22 |
| 16 | C | GTACACTGGCACTACGACCTACAC | 24 | AGAATCGGCCTAGGACAATAGAGTT | 25 |
| 17 | C | AGTTTCTGCACTTGACAACACAAC | 26 | AACTCAGTGATTGTTTCTGCTTTAAC | 27 |
| 18 | C | CAGCCACGTACTACTTCTCTGATCT | 28 | TAGTGTAGGTTGTTGTACCAGTGTACG | 29 |
| 19 | C | GTACACAAGGACAGTATTGTGGGAG | 30 | ACTGGTAGTGTAGGTTGTTGTACCAG | 31 |
| 20 | C | CAGCCACGTACTACTTCTCTGATCT | 28 | CTGGTAGTGTAGGTTGTTGTACCAGT | 32 |
| 21 | C | CACCTACTACTTCTCTGACCTGGAG | 33 | CTTCTGAGCTAACACTTGAAGGTCAC | 34 |
| 22 | O | CACCTACTACTTCTCTGACCTGGAG | 33 | AGAGTTCTTTCTGCCTTCTGAGCTA | 35 |
| 23 | O | CACCTACTACTTCTCTGACCTGGAG | 33 | AGTTCTTTCTGCCTTCTGAGCTAAC | 36 |
| 24 | O | CTGTGACCAATGTGAGAGGTGAC | 37 | ACAATCTTTTGTTTGTGTCTAGCTTC | 38 |
| 25 | O | TGTGAGAGGTGACCTACAAGTGTT | 39 | GTCTTCTGTTTGTTTCTGGCTTC | 40 |
| 26 | O | TCATCATGGACAGATTTGTGAAAGT | 41 | GTCTCCCTCGTGTTTTACTGCTATC | 42 |
| 27 | O | ATCATGGACAGATTTGTGAAAGTGA | 43 | CTCCCTCGTGTTTTACTGCTATCTC | 44 |
| 28 | O | AGATTTGTGAAGATTGGAACCACTA | 45 | GAGTACTTGTTCGTCCCGTTGTA | 46 |
| 29 | O | CAACTTCCTGCCTCTTTCAATTT | 47 | CAATGATCTTCTGTTTGTGTCTGTC | 5 |
| 30 | O | ACTTACTACTTCGCTGATTTAGAAGTGG | 48 | CTAGCACCTGGAGATCACCTCTC | 49 |
| 31 | O | CTTACTACTTCGCTGATTTAGAAGTGG | 50 | ACCGTAGTTAAAGGAGGTAGGCA | 51 |
| 32 | O | GAGTTGCAAGTACAGCAGAGTTGAG | 52 | CAAGAGTTGTTTCATAGGTGCCA | 53 |
| 33 | O | AGTTGCAAGTACAGCAGAGTTGAG | 54 | CAAGAGTTGTTTCATAGGTGCCA | 53 |
| 34 | O | CTTTGATAGCAGTAAAAGGAGACGTT | 55 | AAGTCTCAAGTTGGGAGCATTTCT | 56 |
| 35 | O | TCTTTGATAGCAGTAAAAGGAGACG | 57 | AGTCTCAAGTTGGGAGCATTTCT | 58 |
| 36 | O | AACACACGGACGTCTCATTCATA | 59 | ACTTCTAAGTCAGCGAAATAGTAAGTGG | 60 |
| 37 | O | ACTTACTATTTCGCTGACTTAGAAGTGG | 61 | GTACTTGCAGTTCCCGTTGTAAA | 62 |
| 38 | O | TACTGCTACTTACTACTTCGCAGACCT | 63 | CTAACACTTGCAGGTCACCTCTC | 64 |
| 39 | O | ACTGCTACTTACTACTTCGCAGACCTA | 65 | CCGTTGTAAACAGTAGCCAACAC | 66 |
| 40 | O | AGATTTGTGAAAGTAACACCAAAAGAC | 67 | CGTTGTAAACAGTAGCCAAGACAC | 68 |
| 41 | O | TTAGACAGATTTGTGAAAGTAACACCA | 69 | GTTGTAAACAGTAGCCAAGACACG | 70 |
| 42 | O | GTTTACAACGGGAACTGCAAGTAT | 71 | ACAATCTTTTGTTTGTGTCTAGCTTC | 38 |
| 43 | O | CGTCAGAAACCTCTTAAAGTGAAAG | 72 | CTCAGTGACGATCAAGTTCTTTG | 73 |
| 44 | O | ATCTCAATTCCTTCCCAAAAGTC | 74 | TGATGTTTGCTTTCTCAATGTACTC | 75 |
| 45 | O | AAAGTGACACCAAAAGACCAAATTA | 76 | CGTTGTAGACAGTAGCCAAAACAC | 77 |
| 46 | O | ACCGTGTCTTGGCTACTGTTTAC | 78 | CTTTTGTTTGTGTCTAGCTTCGCT | 79 |
| 47 | O | TTACTCGACTTGCCTTGCCTTAC | 80 | CTATCTTCTGTTTGTGCCTGGCT | 81 |
| 48 | O | CGTCAGAAACCTCTTAAAGTGAAAG | 72 | CTCAGTGACTATCAAGTTCTTTGCT | 82 |
| 49 | O | AGAAACCTCTTAAAGTGAAAGCCAG | 83 | CTCAGTGACTATCAAGTTCTTTGCTT | 84 |
| 50 | O | TTAGACTTGCTCAAGACAAAAGAGAA | 85 | TTGTACTTGCAATCACCGTTGTAG | 86 |
| 51 | SAT1 | GTTAGACTTGCTCAAGACAAAAGAGA | 87 | TTGTACTTGCAATCACCGTTGTAG | 86 |
| 52 | SAT1 | GTTAGACTTGCTCAAGACAAAAGAGA | 87 | CTTGTACTTGCAATCACCGTTGTA | 88 |
| 53 | SAT1 | ACAACAAGATGGTGTTAGACTTGCT | 89 | GTACTTGCAATCACCGTTGTAGGT | 90 |
| 54 | SAT1 | CAGGTGTCTTGCAACAACTTACAAT | 91 | AGGTTTTGTTATCGCTGTCTTGTAG | 92 |
| 55 | SAT1 | AGGTGTCTTGCAACAACTTACAATG | 93 | GTAAATCCTGCCGTAGTTAAAAGTG | 94 |
| 56 | SAT1 | ACACAGGTGTCTTGCAACAACTTAC | 95 | AGGTTTTGTTATCGCTGTCTTGTAG | 92 |
| 57 | SAT1 | TGGTGACTGTAAGTACAAACCCACT | 96 | CAGCTTTAACAGGTCGAAATTACA | 97 |
| 58 | SAT1 | CAACATCCTACAATGGTGACTGTAA | 98 | TACACAACTGTTTGACAGGCTTAGTT | 99 |
| 59 | SAT1 | GTCTTCTCCAAAAACAACACCAC | 100 | GTGTGTAAAGCCTGCCATAGTTAAAG | 101 |
| 60 | SAT1 | AGTCGTCTTCTCCAAAAACAACA | 102 | GTGTGTAAAGCCTGCCATAGTTAAAG | 101 |
| 61 | SAT1 | ACTTTCAACTACGGTAGGATCTACACA | 103 | AACTTTAACAGGTCGAAGTTGCAC | 104 |
| 62 | SAT1 | CTTTCAACTACGGTAGGATCTACACAG | 105 | AACTTTAACAGGTCGAAGTTGCAC | 104 |
| 63 | SAT1 | TCCTACAACGGTGACTGCAAGTA | 106 | GTGATCGTAGTGTGTGAGAAGAGGT | 107 |
| 64 | SAT1 | AACTGTCTACAACGGTGAGTGTAAAT | 108 | TCATCCTGTAGTACACGTCAACACT | 109 |
| 65 | SAT2 | AACTGTCTACAACGGTGAGTGTAAAT | 108 | CATCCTGTAGTACACGTCAACACTT | 110 |
| 66 | SAT2 | TGAGAGCTTCCACCTACTACTTCTG | 111 | TGTGTATTTACACTCACCGTTGTAGA | 112 |
| 67 | SAT2 | CACTGTTTACAACGGTGAGTGTAAGTA | 113 | GCATAGTTGTTTCTCTACCCCAATA | 114 |
| 68 | SAT2 | CACTGTTTACAACGGTGAGTGTAAG | 115 | GCATAGTTGTTTCTCTACCCCAATAG | 116 |
| 69 | SAT2 | TAACACAACTGTACAACCAATACGTG | 117 | GCATAGTTGTTTCTCTACCCCAATA | 114 |
| 70 | SAT2 | CCACCTACTATTTTTGTGACTTGGA | 118 | TGGAGTAGTTACACTCACCGTTGTA | 119 |
| 71 | SAT2 | TATTTTTGTGACTTGGAAATTGCAT | 120 | TGGAGTAGTTACACTCACCGTTGTA | 119 |
| 72 | SAT2 | AGCACTTTCAACTACGGTTACGTG | 121 | ACACAGTTGTTTTTCTACGCCAAT | 122 |
| 73 | SAT2 | GTACTACTTTGCTGACCTTGAAATCG | 123 | ACGTAACCGTAGTTGAAAGTGCTG | 124 |
| 74 | SAT2 | GTTTACAACGGTGAATGCAAATAC | 125 | ATCCTGTAGTACACATCAACGCTACT | 126 |
| 75 | SAT2 | GTTTACAACGGTGAATGCAAATAC | 125 | TGTAGTACACATCAACGCTACTGTCA | 127 |
| 76 | SAT2 | CAAAGCAGTTGATGTGTACTACAGG | 128 | CAACTTTAACAGGTCGTAGTTGCAC | 129 |
| 77 | SAT2 | ACATTCAACTTTGGTCACGTTACTG | 130 | CAACTTTAACAGGTCGTAGTTGCAC | 129 |
| 78 | SAT2 | CCAGCACTTTCAACTACGGTTAC | 131 | ACACAGTTGTTTTTCTACGCCAAT | 122 |
| 79 | SAT2 | GCACTTTCAACTACGGTTACGTG | 132 | ACACAGTTGTTTTTCTACGCCAAT | 122 |
| 80 | SAT2 | GTTTACAACGGTGAATGCAAATAC | 125 | CAACTTTAACAGGTCGAAGTTACACA | 133 |
| 81 | SAT2 | CTATCAACCAGATACAACGGTGAGT | 134 | AGGAGAGGTCTTGGACAGTAGAGTTC | 135 |
| 82 | SAT2 | CTGTCTACTGTCTACAATGGCGAGT | 136 | CTTCATCCGGTAGTAAACATCGACT | 137 |
| 83 | SAT2 | AGGCTGCTGTCTACTGTCTACAATG | 138 | CTTCATCCGGTAGTAAACATCGACT | 137 |
| 84 | SAT2 | AGGCTGCTGTCTACTGTCTACAATG | 138 | ACAGTACAGTTCAGCCCTCTTCAT | 139 |
| 85 | SAT2 | CAGTGTTCTTGCAACAGTCTACAAT | 140 | AATTACACAGTTGTTTATCAGGTGCTAC | 141 |
| 86 | SAT3 | GTCTACAATGGCAACTGCAAATACT | 142 | GTTCTCTTCATCCGGTAGTAGACCT | 143 |
| 87 | SAT3 | GTCTACAATGGCAACTGCAAATACT | 142 | TTCTCTTCATCCGGTAGTAGACCTC | 144 |
| 88 | SAT3 | GTCTACAATGGCAACTGCAAATACT | 142 | ACTTCAACAGGTCGAAATTACACAG | 145 |
| 89 | SAT3 | CTGTAAAGGCTGACACCATCACT | 146 | TCAGGTGCAATGATCTTCTGTTTAC | 147 |
| 90 | Asia | CTGTAAAGGCTGACACCATCACT | 146 | CAGGTGCAATGATCTTCTGTTTAC | 148 |
| 91 | Asia | GTTCTTGACAGGTTTGTGAAACTCA | 149 | GATCAAAAGCTCAGTGATGGTGT | 150 |
| 92 | Asia | GTTCTTGACAGGTTTGTGAAACTCA | 149 | ATCAAAAGCTCAGTGATGGTGTC | 151 |
| 93 | Asia | ACCTCTTTCAACTACGGTGCTGT | 152 | CATAATCTGCTTCTCAGGTGCAA | 153 |
| 94 | Asia | TCTTGACAGGTTTGTGAAACTCACT | 154 | GATCAAAAGCTCAGTGATGGTGT | 150 |
| 95 | Asia | TTTCAACTACGGTGCTGTAAAGG | 155 | CATAATCTGCTTCTCAGGTGCAA | 153 |
| 96 | Asia | GTTCTTGACAGGTTTGTGAAACTC | 156 | GATCAAAAGCTCAGTGATGGTGT | 150 |
| 97 | Asia | GTTCTTGACAGGTTTGTGAAACTC | 156 | ATCAAAAGCTCAGTGATGGTGTC | 151 |
| 98 | Asia | CTTGACAGGTTTGTGAAACTCACT | 157 | GATCAAAAGCTCAGTGATGGTGT | 150 |
| 99 | Asia | CTAGACAACCAGACCAATCCAACT | 158 | CTGAGTAGTGTCAAGAGCTAGCAAAG | 159 |
| 100 | Asia | TAGACAACCAGACCAATCCAACT | 160 | CTGAGTAGTGTCAAGAGCTAGCAAAG | 159 |
| 101 | Asia | CTAGACAACCAGACCAATCCAAC | 161 | CTGAGTAGTGTCAAGAGCTAGCAAAG | 159 |
| 102 | Asia | ACAGTGTACAATGGGAAGACGAC | 162 | CTGAGTAGTGTCAAGAGCTAGCAAAG | 159 |
| 103 | Asia | CTCTAGACAACCAGACCAATCCA | 163 | CTGAGTAGTGTCAAGAGCTAGCAAAG | 159 |
| 104 | Asia | TCTAGACAACCAGACCAATCCAAC | 164 | CTGAGTAGTGTCAAGAGCTAGCAAAG | 159 |
| 105 | Asia | CTCTAGACAACCAGACCAATCCAA | 165 | CTGAGTAGTGTCAAGAGCTAGCAAAG | 159 |
| 106 | Asia | TCTAGACAACCAGACCAATCCAA | 166 | CTGAGTAGTGTCAAGAGCTAGCAAAG | 159 |
| 107 | Asia | GCTCTAGACAACCAGACCAATCC | 167 | CTGAGTAGTGTCAAGAGCTAGCAAAG | 159 |
| 108 | Asia | CTAGACAACCAGACCAATCCAACT | 158 | AGTAGTGTCAAGAGCTAGCAAAGGC | 168 |
| 109 | Asia | ATGCTCTAGACAACCAGACCAATC | 169 | AGTAGTGTCAAGAGCTAGCAAAGGC | 168 |
| 110 | Asia | GATGCTCTAGACAACCAGACCAAT | 170 | AGTAGTGTCAAGAGCTAGCAAAGGC | 168 |
| 111 | Asia | TAGACAACCAGACCAATCCAACT | 160 | AGTAGTGTCAAGAGCTAGCAAAGGC | 168 |
| 112 | Asia | CTAGACAACCAGACCAATCCAAC | 161 | AGTAGTGTCAAGAGCTAGCAAAGGC | 168 |
| 113 | Asia | CTCTAGACAACCAGACCAATCCA | 163 | AGTAGTGTCAAGAGCTAGCAAAGGC | 168 |
| 114 | Asia | TCTAGACAACCAGACCAATCCAAC | 164 | AGTAGTGTCAAGAGCTAGCAAAGGC | 168 |
| 115 | Asia | TGCTCTAGACAACCAGACCAATC | 171 | AGTAGTGTCAAGAGCTAGCAAAGGC | 168 |
| 116 | Asia | GATGCTCTAGACAACCAGACCAA | 172 | AGTAGTGTCAAGAGCTAGCAAAGGC | 168 |
| 117 | Asia | CTCTAGACAACCAGACCAATCCAA | 165 | AGTAGTGTCAAGAGCTAGCAAAGGC | 168 |
| 118 | Asia | ATGCTCTAGACAACCAGACCAAT | 173 | AGTAGTGTCAAGAGCTAGCAAAGGC | 168 |
| 119 | Asia | TCTAGACAACCAGACCAATCCAA | 166 | AGTAGTGTCAAGAGCTAGCAAAGGC | 168 |
| 120 | Asia | AAAGATGCTCTAGACAACCAGACC | 174 | GATCAAAAGCTCAGTAATGGTGTCA | 175 |
| 121 | Asia | CTAGACAACCAAACTAACCCAACTG | 176 | GAGTGGTGTCAAGAGCTAGCAAAG | 177 |
| 122 | Asia | CAAAGATGCTCTAGACAACCAGAC | 178 | GATCAAAAGCTCAGTAATGGTGTCA | 175 |
| 123 | Asia | CCTAGACAACCAAACTAACCCAACT | 179 | GAGTGGTGTCAAGAGCTAGCAAAG | 177 |
| 124 | Asia | ATATGGCTGCCCTTACACTAAAGAC | 180 | GAGTGGTGTCAAGAGCTAGCAAAG | 177 |
| 125 | Asia | GTGTACTGGCGACAGTGTACAAG | 181 | GATCAAAAGCTCAGTAATGGTGTCA | 175 |
| 126 | Asia | ATATGGCTGCCCTTACACTAAAGA | 182 | GAGTGGTGTCAAGAGCTAGCAAAG | 177 |
| 127 | Asia | GATATGGCTGCCCTTACACTAAAG | 183 | GAGTGGTGTCAAGAGCTAGCAAAG | 177 |
| 128 | Asia | GTGTACTGGCGACGGTATACAAC | 184 | GATCAAAAGCTCAGTAATGGTGTCA | 175 |
| 129 | Asia | TATGGCTGCCCTTACACTAAAGACT | 185 | GAGTGGTGTCAAGAGCTAGCAAAG | 177 |
| 130 | Asia | AATCAGACCAATCCAACTGCTTAC | 186 | GAGTGGTGTCAAGAGCTAGCAAAG | 177 |
| 131 | Asia | CTAGACAACCAGACCAATCCAACT | 158 | GAGTGGTGTCAAGAGCTAGCAAAG | 177 |
| 132 | Asia | GACAGTGTACAACGGGAAGACTAC | 187 | GAGTGGTGTCAAGAGCTAGCAAAG | 177 |
| 133 | Asia | TATGGCTGCCCTTACACTAAAGAC | 188 | GAGTGGTGTCAAGAGCTAGCAAAG | 177 |
| 134 | Asia | GATATGGCTGCCCTTACACTAAAGA | 189 | GAGTGGTGTCAAGAGCTAGCAAAG | 177 |
| 135 | Asia | AATCAGACCAATCCAACTGCTTA | 190 | GAGTGGTGTCAAGAGCTAGCAAAG | 177 |
| 136 | Asia | TAGACAACCAGACCAATCCAACT | 160 | GAGTGGTGTCAAGAGCTAGCAAAG | 177 |
| 137 | Asia | CTAGACAACCAGACCAATCCAAC | 161 | GAGTGGTGTCAAGAGCTAGCAAAG | 177 |
| 138 | Asia | TATGGCTGCCCTTACACTAAAGA | 191 | GAGTGGTGTCAAGAGCTAGCAAAG | 177 |
| 139 | Asia | GTGTACTGGCGACAGTGTACAAG | 181 | GAGTGGTGTCAAGAGCTAGCAAAG | 177 |
| 140 | Asia | AGACTACGTACGGGGAAACAACT | 192 | GAGTGGTGTCAAGAGCTAGCAAAG | 177 |
| 141 | Asia | AAGACTACGTACGGGGAAACAACT | 193 | GAGTGGTGTCAAGAGCTAGCAAAG | 177 |
| 142 | Asia | ATCAGACCAATCCAACTGCTTAC | 194 | GAGTGGTGTCAAGAGCTAGCAAAG | 177 |
| 143 | Asia | AGACTACGTACGGGGAAACAACTT | 195 | GAGTGGTGTCAAGAGCTAGCAAAG | 177 |
| 144 | Asia | GATATGGCTGCCCTTACACTAAA | 196 | GAGTGGTGTCAAGAGCTAGCAAAG | 177 |
| 145 | Asia | CTCTAGACAACCAGACCAATCCA | 163 | GAGTGGTGTCAAGAGCTAGCAAAG | 177 |
| 146 | Asia | ACAGTGTACAACGGAAAGACGAC | 197 | GAGTGGTGTCAAGAGCTAGCAAAG | 177 |
| 147 | Asia | TCTAGACAACCAGACCAATCCAAC | 164 | GAGTGGTGTCAAGAGCTAGCAAAG | 177 |
| 148 | Asia | ACTACGTACGGGGAAACAACTTC | 198 | GAGTGGTGTCAAGAGCTAGCAAAG | 177 |
| 149 | Asia | AAGACTACGTACGGGGAAACAAC | 199 | GAGTGGTGTCAAGAGCTAGCAAAG | 177 |
| 150 | Asia | AACAGTGTACAACGGAAAGACGAC | 200 | GAGTGGTGTCAAGAGCTAGCAAAG | 177 |
| 151 | Asia | GACTACGTACGGGGAAACAACTT | 201 | GAGTGGTGTCAAGAGCTAGCAAAG | 177 |
| 152 | Asia | AAAGATGCTCTGGACAACCAAAC | 202 | GATCAAAAGCTCAGTAATGGTGTCA | 175 |
| 153 | Asia | ATGGCTGCCCTTACACTAAAGACT | 203 | GAGTGGTGTCAAGAGCTAGCAAAG | 177 |
| 154 | Asia | ACAGTGTACAACGGGAAGACTACG | 204 | GAGTGGTGTCAAGAGCTAGCAAAG | 177 |
| 155 | Asia | CTCTAGACAACCAGACCAATCCAA | 165 | GAGTGGTGTCAAGAGCTAGCAAAG | 177 |
| 156 | Asia | ATATGGCTGCCCTTACACTAAAGACT | 205 | GAGTGGTGTCAAGAGCTAGCAAAG | 177 |
| 157 | Asia | ATGGCTGCCCTTACACTAAAGAC | 206 | GAGTGGTGTCAAGAGCTAGCAAAG | 177 |
| 158 | Asia | TCTAGACAACCAGACCAATCCAA | 166 | GAGTGGTGTCAAGAGCTAGCAAAG | 177 |
| 159 | Asia | AACAGTGTACAACGGAAAGACGA | 207 | GAGTGGTGTCAAGAGCTAGCAAAG | 177 |
| 160 | Asia | TGATATGGCTGCCCTTACACTAAA | 208 | GAGTGGTGTCAAGAGCTAGCAAAG | 177 |
| 161 | Asia | GCAACAGTGTACAACGGAAAGAC | 209 | GAGTGGTGTCAAGAGCTAGCAAAG | 177 |
| 162 | Asia | CAGTGTACAACGGGAAGACTACG | 210 | GAGTGGTGTCAAGAGCTAGCAAAG | 177 |
| 163 | Asia | ATATGGCTGCCCTTACACTAAAG | 211 | GAGTGGTGTCAAGAGCTAGCAAAG | 177 |
| 164 | Asia | GTGTACAACGGAAAGACGACGTA | 212 | GAGTGGTGTCAAGAGCTAGCAAAG | 177 |
| 165 | Asia | GACAGTGTACAACGGAAAGACGA | 213 | GAGTGGTGTCAAGAGCTAGCAAAG | 177 |
| 166 | Asia | TGGCTGCCCTTACACTAAAGACT | 214 | GAGTGGTGTCAAGAGCTAGCAAAG | 177 |
| 167 | Asia | GAAGACTACGTACGGGGAAACAA | 215 | GAGTGGTGTCAAGAGCTAGCAAAG | 177 |
| 168 | Asia | AAGACGACGTACGGAAAACAAAC | 216 | GAGTGGTGTCAAGAGCTAGCAAAG | 177 |
| 169 | Asia | GACAGTGTACAACGGGAAGACTA | 217 | GAGTGGTGTCAAGAGCTAGCAAAG | 177 |
| 170 | Asia | ATCCAACTGCCTACCAGAAACAG | 218 | GAGTGGTGTCAAGAGCTAGCAAAG | 177 |
| 171 | Asia | ACAGTGTACAAGGGGAAGACGAC | 219 | GAGTGGTGTCAAGAGCTAGCAAAG | 177 |
| 172 | Asia | CAACAGTGTACAACGGAAAGACG | 220 | GAGTGGTGTCAAGAGCTAGCAAAG | 177 |
| 173 | Asia | AAAGATGCTCTAGACAACCAGACC | 174 | ATCAAAAGCTCAGTAATGGTGTCAG | 221 |
| 174 | Asia | ATGCTCTAGACAACCAGACCAAC | 222 | ATCAAAAGCTCAGTAATGGTGTCAG | 221 |
| 175 | Asia | GTGTACTGGCGACAGTGTACAAG | 181 | ATCAAAAGCTCAGTAATGGTGTCAG | 221 |
| 176 | Asia | AGATGCTCTAGACAACCAGACCAA | 223 | ATCAAAAGCTCAGTAATGGTGTCAG | 221 |
| 177 | Asia | GATGCTCTAGACAACCAGACCAA | 172 | ATCAAAAGCTCAGTAATGGTGTCAG | 221 |
| 178 | Asia | GTGTACTGGCGACGGTATACAAC | 184 | ATCAAAAGCTCAGTAATGGTGTCAG | 221 |
| 179 | Asia | CCAAAGATGCTCTAGACAACCAGA | 224 | ATCAAAAGCTCAGTAATGGTGTCAG | 221 |
| 180 | Asia | ACTGACTACCAGAAGCAACCCAT | 225 | ATCAAAAGCTCAGTAATGGTGTCAG | 221 |
| 181 | Asia | CTAGACAACCAAACTAACCCAACTG | 176 | GAGTAGTGTCAAGAGCTAGCAAAGG | 226 |
| 182 | Asia | CTAGACAACCAGACCAATCCAACT | 158 | GAGTAGTGTCAAGAGCTAGCAAAGG | 226 |
| 183 | Asia | ATGCTCTAGACAACCAGACCAATC | 169 | GAGTAGTGTCAAGAGCTAGCAAAGG | 226 |
| 184 | Asia | TAGACAACCAGACCAATCCAACT | 160 | GAGTAGTGTCAAGAGCTAGCAAAGG | 226 |
| 185 | Asia | CTAGACAACCAGACCAATCCAAC | 161 | GAGTAGTGTCAAGAGCTAGCAAAGG | 226 |
| 186 | Asia | CTAGACAACCAGACCAATCCAACT | 158 | TGAGTAGTGTCAAGAGCTAGCAAAG | 227 |
| 187 | Asia | ACAGTGTACAATGGGAAGACGAC | 162 | GAGTAGTGTCAAGAGCTAGCAAAGG | 226 |
| 188 | Asia | TAGACAACCAGACCAATCCAACT | 160 | TGAGTAGTGTCAAGAGCTAGCAAAG | 227 |

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A composition comprising a mixture, wherein said mixture comprises at least one primer pair selected from the group consisting of primer pair numbers 1, 2, 3, 4, 5, 6, 7, 8, 9, and 10, wherein said primer pair is capable of amplifying a sequence present in a foot-and-mouth disease virus.

2. The composition of claim 1, wherein said mixture is a solid.

3. The composition of claim 1, wherein said mixture is a liquid.

4. An article of manufacture comprising (a) a substrate defining a microfluidic chamber and (b) a mixture comprising at least one primer pair selected from the group consisting of primer pair numbers 1, 2, 3, 4, 5, 6, 7, 8, 9, and 10; wherein said mixture is within said chamber; wherein said primer pair is capable of amplifying, within said chamber, a sequence present in a foot-and-mouth disease virus.

5. The article of manufacture of claim 4, wherein said mixture is a solid.

6. The article of manufacture of claim 4, wherein said mixture is a liquid.

7. A diagnostic card for determining whether or not a cow contains a foot-and-mouth disease virus, wherein said card comprises a plurality of microfluidic chambers, wherein at least one of said microfluidic chambers comprises at least one primer pair selected from the group consisting of primer pair number 1, 2, 3, 4, 5, 6, 7, 8, 9, and 10, which are capable of amplifying, within said chamber, a sequence present in a foot-and-mouth disease virus.

8. A method for determining whether or not a cloven-hooved animal contains a foot-and-mouth disease virus, wherein said method comprises performing an amplification reaction with at least one primer pair selected from the group consisting of primer pair numbers 1, 2, 3, 4, 5, 6, 7, 8, 9, and 10 to determine whether or not a sample from said animal contains nucleic acid capable of being amplified with said primer pair, wherein the presence of said nucleic acid indicates that said animal contains a foot-and-mouth disease virus.

9. The method of claim 8, wherein said animal is a cow.

10. The method of claim 8, wherein said sample is a blood sample.

11. A method for making an article of manufacture for determining whether or not a cloven-hooved animal contains a foot-and-mouth disease virus, said method comprising:
(a) providing a substrate defining a microfluidic chamber, and
(b) placing a mixture into said chamber to form said article of manufacture,
wherein said mixture comprises at least one primer pair selected from the group consisting of primer pair numbers 1, 2, 3, 4, 5, 6, 7, 8, 9, and 10; wherein said mixture is within said chamber; wherein said primer pair is capable of amplifying, within said chamber, a sequence present in a foot-and-mouth disease virus.

12. The method of claim 11, wherein said mixture is a solid.

13. The method of claim 11, wherein said mixture is a liquid.

14. The method of claim 11, wherein said animal is a cow.
